# FASCICULE DE BREVET EUROPEEN

(11) **EP 1 326 635 B1**
(45) Date de publication et mention de la délivrance du brevet: **11.05.2005**
(21) Numéro de dépôt: 01976378.8
(22) Date de dépôt: 08.10.2001
(51) Int. Cl.: A61K 39/21

(54) **COMPOSITION PHARMACEUTIQUE POUR L'IMMUNISATION CONTRE LE SIDA**
ZUBEREITUNG ZUR IMMUNISIERUNG GEGEN DEN AIDS-VIRUS
PHARMACEUTICAL COMPOSITION FOR IMMUNISATION AGAINST AIDS

(30) Priorité: 06.10.2000 FR 0012808
(43) Date de publication de la demande: 16.07.2003
(73) Titulaire: Sanofi Pasteur, 69367 Lyon Cedex 07 (FR)
(72) Inventeur: HAENSLER, Jean, F-69290 St Genis les Ollières (FR); DALENCON, François, F-69004 Lyon (FR)
(74) Mandataire: Kerneis, Danièle
(86) Numéro de dépôt international: PCT/FR2001/003096
(87) Numéro de publication internationale: WO 2002/028427

(56) Documents cités:
- WO-A-01/15726
- FARHOOD, HASSAN ET AL: "Codelivery to mammalian cells of a transcriptional factor with cis-acting element using cationic liposomes" ANALYTICAL BIOCHEMISTRY (1995), 225(1), 89-93 , XP001126233
- FARHOOD H ET AL: "Cationic liposomes for direct gene transfer in therapy of cancer and othe diseases." ANNALS OF THE NEW YORK ACADEMY OF SCIENCES, (1994 MAY 31) 716 23-34;DISCUSSION 34-5. REF: 29 , XP001122855

## Description

La présente invention est relative au domaine des compositions pharmaceutiques utilisable pour l'immunisation contre les infections liées au VIH.

Le syndrome de l'immunodéficience acquise ou SIDA est une maladie contre laquelle il serait très souhaitable de pouvoir disposer d'un vaccin, et en particulier d'un vaccin utilisable en prophylaxie. Or, jusqu'à aujourd'hui, le développement d'un tel vaccin s'est heurté à de nombreux problèmes dont notamment les formes diverses que peut prendre le virus VIH, ainsi que les difficultés à identifier les corrélats de l'immunité contre le VIH, c'est-à-dire les facteurs immunitaires susceptibles de protéger contre les virus. Les cas de personnes exposées et non infectées ainsi que les études sur les « non-progresseurs » donnent cependant des indications, et orientent les recherches vers l'induction de 2 types de réponse immunitaire : la réponse par les anticorps (humorale) et la réponse cellulaire.
La réponse humorale qu'il est souhaitable de susciter est non seulement une réponse humorale systémique, mais également une réponse muqueuse car il est souhaitable de pouvoir dresser une barrière immunitaire contre le virus sur le lieu de son entrée dans l'organisme.
Les stratégies de recherche d'un vaccin contre le SIDA s'orientent donc vers la recherche de compositions vaccinales capables d'inciter le système immunitaire à produire notamment une forte quantité d'Ig G et d'Ig A spécifiques du virus du SIDA, en particulier dans les muqueuses.
Cet objectif se heurte cependant à de nombreuses difficultés, car les antigènes du VIH, susceptibles d'être utilisés dans une composition vaccinale, ne sont pas toujours capables d'induire, à eux seuls, une réponse suffisante, et les formulations connues dans l'art antérieur pour leur capacité à induire une telle réponse, telles que les formulations comprenant la toxine cholérique, présentent parfois des risques de toxicité non négligeables.

Le but de la présente invention est donc de proposer une nouvelle composition pharmaceutique susceptible d'être utilisée pour l'immunisation, à titre prophylactique ou thérapeutique, contre les infections liées au VIH. De façon surprenante, on a mis en évidence que la composition selon l'invention permet d'induire une production importante d'Ig G et d'Ig A spécifiques d'un antigène du VIH.

Pour atteindre ce but, la présente invention propose l'utilisation d'une composition comprenant au moins un antigène du VIH et du DCchol, pour la fabrication d'un médicament pour l'immunisation contre les infections liées au VIH, destiné à être administré par voie muqueuse.

Selon une caractéristique de l'invention le médicament est destiné à l'induction dans les tissus muqueux d'un mammifère d'anticorps Ig G et Ig A spécifiques d'un antigène du VIH.

D'autres caractéristiques et avantages de la présente invention apparaîtront à la lecture de la description qui suit, en référence aux dessins qui illustrent des résultats obtenus.
Les figures 1 à 3 illustrent les résultats obtenus à l'exemple 5.
La figure 4 illustre les résultats obtenus à l'exemple 6.
Les figures 5 et 6 illustrent les résultats obtenus à l'exemple 7.
Les figures 7 à 13 illustrent les résultats obtenus à l'exemple 8.

Par antigène de VIH, on entend tout antigène susceptible d'induire des anticorps neutralisants issus du VIH 1 ou 2, de préférence issu du VIH 1, et incluant les souches de laboratoire et les isolats primaires. Ce terme englobe donc notamment les antigènes de surface du virus tels que la glycoprotéine 160 ou gp160, ou les protéines en dérivant i.e. gp120 et gp41, les antigènes constitués par des protéines internes telles que la protéine Gag et les protéines en dérivant telles que p24, p17, ou encore les antigènes issus des protéines de régulation tels que la protéine Tat. La liste donnée ci-dessus n'est pas limitative. Par antigène du VIH au sens de l'invention, on entend également les antigènes tels que définis ci-dessus qui ont été modifiés par traitement chimique ou génétiques pour autant que le traitement appliqué n'altère pas substantiellement les propriétés immunologiques de l'antigène.

On peut citer à titre d'exemple les protéines de régulation du VIH détoxifiées par voie chimique telles que décrites dans les brevets ou demandes de brevet suivant : US6200575, US6132721, WO99/33346 ; la protéine Tat detoxifiée par mutation telle que décrite par Golstein dans la demande WO95/31999 et Nature Medicine, 1, 960, (1996) et par Loret dans la demande WO00/61067. De préférence la protéine Tat utilisée dans le cadre de la présente invention est une protéine dépourvue de son activité de transactivation et de son activité immunosuppressive.

La suppression de l'activité de transactivation peut être aisément contrôlée par le test CAT tel que décrit par G. Tosi et al dans Eur. J. Immuno. (2000) 30, pp1120 -1126. La suppression de l'acitvité immunosuppression de la protéine Tat peut être aisément déterminée par le test d'immunosuppression tel que décrit par Zagury dans Proc. Natl. Acad. Sci. USA 1998, 95 : 3851-6.

Par antigène du VIH au sens de l'invention, on entend donc l'antigène lui-même, mais également l'ADN qui, après administration au moyen d'un vecteur tel qu'un canarypox, est susceptible d'être exprimé par l'organisme et donc de conduire à la production in situ de l'antigène contre lequel on souhaite induire une réponse immunitaire. La présente invention englobe donc outre les antigènes protéiques définis ci-dessus les vecteurs, de préférence viraux exprimant ces antigènes. Les vecteurs viraux utilisés de préférence dans le cadre de la présente invention sont les vecteurs ALVAC et NYVAC qui sont décrits en détails dans les brevets US5364773, US5756103, US5990091 auxquels on pourra se référer pour un descriptif complet de ces vecteurs et de leur procédé d'obtention. L'antigène peptidique selon l'invention peut être produit par synthèse chimique ou par génie génétique. Les séquences ADN et protéiques d'un grand nombre d'antigène du VIH sont disponibles sur le site : http://hiv-web.lanl.gov/ et dans les compendium de Los Adamos correspondants.

Lorsque l'antigène est produit par synthèse chimique, l'antigène selon l'invention peut être synthétisé sous la forme d'une séquence unique, ou sous la forme de plusieurs séquences qui sont ensuite liées les unes aux autres. La synthèse chimique peut être réalisée en phase solide ou en solution, ces deux techniques de synthèse étant bien connues de l'homme de l'art. Ces techniques sont décrites notamment par Atherton et Shepard dans « solid phase peptide synthesis (IRL press Oxford, 1989) et par Houbenweyl dans « method der organischen chemie » édité par E.Wunsch vol, 15-I et II thieme, Stuttgart, 1974, Dawson PE et al (Science 1994 ; 266(5186):776-9);Kochendoerfer GG et al (Curr Opin Chem Biol 1999 ;3(6):665-71); et Dawson PE et al (Annu Rev Biochem 2000 ; 69:923-60) auxquels on pourra se référer pour un descriptif complet des techniques de synthèse.

L'antigène selon l'invention peut être également produit par les techniques de génie génétique bien connues de l'homme de l'art. Ces techniques sont décrites en détails dans Molecular Cloning: a molecular manual de Maniatis et al, Cold Spring Harbor, 1989). Classiquement, la séquence d'ADN codant pour le polypeptide selon l'invention peut être produite par la technique de PCR dans laquelle les séquences N et C sont dans un premier temps amplifiées indépendamment l'une de l'autre puis sont dans un deuxième temps appariées et de nouveau amplifiées. La séquence d'ADN ainsi obtenue est ensuite insérée dans un vecteur d'expression. Le vecteur d'expression renfermant la séquence d'intérêt est alors utilisé pour transformer une cellule hôte permettant l'expression de la séquence d'intérêt. Le polypeptide produit est ensuite isolé du milieu de culture par des techniques classiques bien connues de l'homme de l'art, telles que précipitation à l'éthanol ou au sulfate d'ammonium, extraction par l'acide, chromatographie d'échange d'anion/cation, chromatographie sur phosphocellulose, chromatographie d'interaction hydrophobe, chromatographie d'affinité, chromatographie sur hydroxyapatite et chromatographie sur lectine. De préférence, la chromatographie liquide à haute performance (HPLC) est utilisée dans la purification.

Pour mettre en oeuvre la synthèse de l'antigène, tout vecteur d'expression classiquement utilisé pour l'expression d'une protéine recombinante peut être utilisé dans le cadre de la présente invention. Ce terme englobe donc aussi bien les vecteurs d'expression dits « vivants » tels que les virus et les bactéries que les vecteurs d'expression de type plasmidiques.

On utilise de préférence des vecteurs dans lesquels la séquence d'ADN de l'antigène selon l'invention est sous la dépendance d'un promoteur fort, inductible ou non inductible. On peut citer à titre d'exemple de promoteur utilisable, le promoteur de l'ARN polymérase T7.

Les vecteurs d'expression incluent de préférence au moins un marqueur de sélection. De tels marqueurs incluent, par exemple, le gène de la dihydrofolate réductase ou le gène de résistance à la néomycine pour la culture des cellules d'eucaryote et les gènes de résistance à la kanamycine, tétracycline ou ampicilline pour la culture dans *E*. *coli* et des autres bactéries.

On peut citer à titre de vecteur d'expression utilisable dans le cadre de la présente invention les plasmides tels que pET28 (Novagen) ou pBAD (Invitrogen) par exemple.

Pour favoriser l'expression et la purification de l'antigène, ce dernier peut être exprimé dans une forme modifiée, telle qu'une protéine de fusion, et peut inclure non seulement des signaux de sécrétion, mais aussi des régions fonctionnelles hétérologues supplémentaires. Par exemple, une région d'acides aminés supplémentaires, particulièrement des acides aminés chargés, peut être ajoutée au N-terminal de l'antigène pour améliorer la stabilité et le maintien dans la cellule hôte.

Pour l'expression de l'antigène, toute cellule hôte classiquement utilisée en association avec les vecteurs d'expression décrits ci-dessus peut être utilisée.

On peut citer à titre d'exemple non limitatif les cellules de E. coli, BL21 (λDE3), HB101, Top 10, CAG 1139, Bacillus, les cellules eucaryotes telles que CHO ou Vero. De préférence on utilisera dans le cadre de la présente invention le système vecteur d'expression /cellule suivant : pET(Cer) /BL21LamdaDE3, ou BL21lamdaDE3(RIL).

On a préparé des compositions pharmaceutiques appropriées pour une immunisation contre le virus de l'immunodéficience humaine de type 1 (VIH-1) dans lesquelles l'antigène est la glycoprotéine d'enveloppe gp160 MN/LAI-2. Cet antigène contient la portion gp120 de l'isolat VIH-1 MN et la portion gp41 de l'isolat VIH-1 LAI. La gp41 a été délétée de son site de clivage avec la gp120 et de sa partie transmembranaire de façon à obtenir une glycoprotéine non clivée et essentiellement sécrétée. L'antigène est produit à partir de la lignée cellulaire de hamster BHK-21 infectée par le virus recombinant de la vaccine VVTG.9150 dérivé de la précédente construction VVTG.1163 (Kieny, M.-P. et al, 1988, Protein Eng, 2(3) : 219-255), puis est purifié par chromatographie d'échange d'ions suivie d'une chromatographie d'immunoaffinité.

On a également préparé des compositions pharmaceutiques comprenant l'antigène p24. Cet antigène p24 a été décrit dans la publication suivante : *Diagnostic value of HIV-Ag testing and anti-p24 titers in HIV carriers and AIDS patients*.Roumeliotou A, Nestoridou E, Economidou I, Psarra E, Sidiri E, Choremi E, Kallinikos G, Papaevangelou G, *AIDS* 1988 Feb;2(1):64.

On a en outre, préparé des compositions pharmaceutiques comprenant comme antigène une protéine Tat III B détoxifiée . La protéine Tat a été détoxifiée par une réaction d'alkylation en milieu alcalin par utilisation d'iodoacétamide dans les conditions suivantes : nombre de micromoles d'iodoacétamide = 200 X nombre de micromoles de Tat + nombre de micromoles de DTT. Cette protéine détoxifiée et son procédé de préparation sont décrits en détails dans la demande WO99/33346 où elle est identifiée sous le terme de Tat carboxyméthylée.

Par DCchol, on entend le produit qui peut être obtenu à partir de cholesteryl chloroformate et de N, N-dimethylethylenediamine, selon la méthode décrite dans le brevet US 5283185 ou de façon préférée, selon la méthode décrite à l'exemple 8 de la demande de brevet WO 96/40067. Il est possible également d'utiliser un produit obtenu par réaction de cholestéryl chloroformate et de N,N,N triméthyléthylènediamine.

Les compositions vaccinales selon l'invention sont susceptibles d'être formulées sous différentes formes, et notamment sous forme d'émulsions, de liposomes, de liposomes en émulsions, de micelles, ... etc. De particulièrement bons résultats ont été obtenus avec des émulsions, en particulier des émulsions microfluidisées comprenant du squalène, et un détergent tel que le Tween® 80.

Selon une caractéristique de l'invention, le DCchol peut également être associé à un oligonucléotide immunostimulant; en effet, on a remarqué qu'on obtenait dans ce cas, une synergie entre les 2 adjuvants. On peut notamment utiliser des oligonucléotides, tels que ceux décrits dans la demande WO 96/02555, ceux décrits dans la demande EP0468520, ceux décrits dan la demande WO0075304 ou tout autre oligonucléotide connu pour son activité immunoadjuvante.
De façon particulière, on peut utiliser l'oligonucléotide 3Db(S), dont la séquence est décrite sous SEQ ID NO 15 dans la demande WO96/02555.

Les compositions pharmaceutiques selon l'invention sont destinées à être administrées par toutes les voies classiquement utilisées en vaccination, et en particulier par voie parentérale, mais également par voie muqueuse, notamment la voie orale, nasale, rectale, vaginale. Elles peuvent être administrés selon différents protocoles , comportant une seule ou plusieurs étapes d'administration.

Dans le cas d'un protocole en plusieurs étapes, il est possible que les compositions selon l'invention soient administrées à chaque étape, ou simplement à certaines d'entre elles. Il est en effet possible qu'un protocole d'administration prévoyant une étape de primo-immunisation suivi d'une étape de rappel, prévoit l'étape de primo-immunisation au moyen d'une composition différente de celle de l'invention, alors que la composition pharmaceutique selon l'invention serait utilisée uniquement pour l'étape de rappel.
Cependant, étant donné les qualités des compositions pharmaceutiques selon l'invention, il est également possible de les utiliser à chacune des étapes proposées.

La quantité d'antigène dans la composition selon la présente invention dépend de nombreux paramètres comme le comprendra l'homme de l'art, tels que la nature de l'antigène, le vecteur utilisé ou, la voie d'administration. Une quantité appropriée est une quantité telle qu'une réponse immunitaire humorale capable de neutraliser des isolats primaires du VIH est induite après administration de cette dernière. La quantité d'antigène protéique à administrer est de l'ordre de 10 à 100 micro grammes. Dans le cas où l'antigène utilisé est un vecteur viral, la quantité de vecteur à administrer est de l'ordre de 10⁴ à 10⁸TCID₅₀.

Les compositions selon la présente invention peuvent être préparées par tout procédé classique connu de l'homme de d'art. Classiquement les antigènes selon l'invention sont mélangés avec un support ou diluant pharmaceutiquement acceptable, tel que eau ou solution saline tamponnée au phosphate. Le support ou diluant va être sélectionné en fonction de la forme galénique choisie, du mode et de la voie d'administration ainsi que de la pratique pharmaceutique. Les supports ou diluants appropriés ainsi que les exigences en matière de formulation pharmaceutique sont décrits en détails dans Remington's Pharmaceutical Sciences, représentant un ouvrage de référence dans ce domaine.

Les exemples qui suivent illustrent des modes de réalisation particuliers de la présente invention.

### Exemple 1

On a utilisé du chorhydrate de DC-Chol (obtenu selon le mode de préparartion décrit à l'exemple 8 de la demande de brevet WO 96/40067) que l'on a mis en suspension à 20 mg/ml dans du tampon TRIS-NaCl (20 mM TRIS, 150 mM NaCl, pH 6.8). Après 8 heures sous agitation à 35-40°C sous argon, la suspension a été microfluidisée à l'aide d'un microfluidiseur M-110S de chez Microfluidics (10 cycles à 500 kPa), afin de générer une suspension homogène de DC-Chol, que l'on a filtrée sur un filtre Millex 0.45 µm.

### Exemple 2

On a préparé des oligonucléotides grâce à un automate synthétiseur fourni par Applied Biosystems qui met en oeuvre la méthode chimique standard au phosphoramidite et qui comporte à chaque cycle une étape d'oxydation.
Cette étape d'oxydation a été réalisée au moyen d'une solution iode/eau/tétrahydrofurane/acétonitrile pour obtenir une liaison phosphodiester et au moyen d'une solution tétraéthylthiuram/acétonitrile pour obtenir une liaison phosphorothioate.

On a ainsi préparé un oligonucléotide 3 Db(S) dont la séquence est reproduite dans la demande de brevet WO96/02555 sous SEQ ID NO 15 et qui comporte des liaisons phosphorothioate sur toute sa longueur.
On a préparé également un oligonucléotide MGC (S) dont la séquence est reproduite dans la demande de brevet WO00/15256 à SEQ ID NO 2, qui comporte à la fois des liaisons phosphodiester et des liaisons phosphorothioate. Les liaisons phosphorothiate sont situées à chaque extrémité ; il y a 2 liaisons phosphorothioate en 3' et 5 liaisons phosphorothioate en 5'. Cet oligonucléotide ne possède pas de séquence CG et est utilisé comme contrôle négatif.

### Exemple 3 : gp160 +oligonucléotide immunostimulant chez la souris

On a préparé des compositions pharmaceutiques pour l'immunisation contre le virus de l'immunodéficience humaine de type 1 (VIH-1) dans lesquelles l'antigène est la glycoprotéine d'enveloppe gp160 MN/LAI-2. Cet antigène contient la portion gp120 de l'isolat VIH-1 MN et la portion gp41 de l'isolat VIH-1 LAI. La gp41 a été délétée de son site de clivage avec la gp120 et de sa partie transmembranaire de façon à obtenir une glycoprotéine non clivée et essentiellement sécrétée. L'antigène est produit à partir de la lignée cellulaire de hamster BHK-21 infectée par le virus recombinant de la vaccine VVTG.9150 dérivé de la précédente construction VVTG.1163 (Kieny, M.-P. et al, 1988, Protein Eng, 2(3) : 219-255), puis est purifié par chromatographie d'échange d'ions suivie d'une chromatographie d'immunoaffinité.

Les doses pour immunisation de 20µl répondaient à l'une des formulations suivantes:
- 25 µg de gp160 uniquement,
- 25 µg de gp160 + 50 µg d'oligonucléotide 3Db(S) préparé à l'exemple 2,
- 25 µg de gp160 + 50 µg d'oligonucléotide MGC préparé à l'exemple 2 + 200 µg de DCchol préparé à l'exemple 1,
- 25 µg de gp160 + 50 µg d'oligonucléotide 3Db(S) préparé à l'exemple 2 + 200 µg de DCchol préparé à l'exemple 1.

On a injecté les doses préparées à 4 groupes de 6 souris (1 formulation par groupe) par voie rectale, sous anesthésie, à raison de 4 injections séparées chacune de 2 semaines ( soient J1, J15, J29 et J44).

A J57, on a prélevé du sérum, on a recueilli les fécès et on a procédé à des lavages rectaux afin d'effectuer les dosages suivants:
- dosage par ELISA des IgG anti-gp 160 dans le sérum,
- dosage par ELISA des IgA et des IgG totales ainsi que des IgA et des IgG spécifiques anti-gp160 dans les lavages rectaux,
- dosage par ELISA des IgA et des IgG totales ainsi que des IgA et des IgG spécifiques anti-gp160 dans les fécès.

La composition pharmaceutique contenant l'oligonucléotide MGC a été considérée comme un contrôle négatif par rapport à l'oligonucléotide 3Db(S). En effet, l'oligonucléotide MGC s'était révélé ne pas être immunostimulant dans des expériences précédentes. Les résultats obtenus sont donc considérés équivalents à ceux obtenus en utilisant du DCchol comme seul adjuvant.

Les résultats obtenus sont récapitulés dans les tableaux ci-après; seules les moyennes par groupe de souris ayant reçu la même composition sont indiquées.

**Tableau 1 :**

| Dosage des IgG spécifiques dans le sérum : | |
|---|---|
| | IgG anti-gp 160 en µg/ml |
| 25 µg gp160 | 60,55 |
| 25 µg gp160 + 50 µg 3Db(S) | 46,97 |
| 25 µg gp160 + DCchol 200µg + 50µg MGC | 47,85 |
| 25 µg gp160 + DCchol 200µg +50µg 3Db(S) | 645,26 |

Ces résultats montrent la synergie exercée par les 2 adjuvants pour la production d'IgG vis-à-vis de l'antigène gp160, lors d'une administration par voie muqueuse.

**Tableau 2:**

| Dosage des IgA et des IgG dans les lavages rectaux: | | |
|---|---|---|
| | IgA spéc./IgA tot en % | IgG spéc./IgG tot. en % |
| 25 µg gp160 | 0,15 | 3,68 |
| 25 µg gp160 + 50 µg 3Db(S) | 0,91 | 2,46 |
| 25 µg gp160 + DCchol 200µg + 50µg MGC | 0,68 | 1,07 |
| 25 µg gp160 + DCchol 200µg +50µg 3Db(S) | 1,53 | 12,43 |

**Tableau3:**

| Dosage des IgA et des IgG dans les fécès: | | |
|---|---|---|
| | IgA spéc./IgA tot. x 10⁴ | IgG spéc./IgG tot en % |
| 25 µg gp160 | 2,44 | 0,00 |
| 25 µg gp160 + 50 µg 3Db(S) | 18,05 | 1,33 |
| 25 µg gp160 + DCchol 200µg + 50µg MGC | 43,38 | 0,00 |
| 25 µg gp160 + DCchol 200µg +50µg 3Db(S) | 104,79 | 3,03 |

Ces résultats montrent l'effet synergique obtenu en utilisant à la fois du DCchol et des oligonucléotides immunostimulants, vis-à-vis de la production locale d'immunoglobulines G et d'immunoglobulines A spécifiques.

Cette capacité à stimuler localement la production d'IgA spécifiques est particulièrement recherchée dans l'immunisation contre les infections liées au VIH, et confirme l'intérêt de l'objet de la présente invention.

### Exemple 4 : gp160 chez les souris

On dispose de gp 160 telle que décrite à l'exemple 3, de DCchol tel que décrit à l'exemple 1, et de toxine cholérique CT fournie par la Société Sigma sous la référence #C-8052.

A partir de ces produits, on prépare des compositions immunisantes, comprenant soit de la gp 160 seule, soit de la gp160 et du DCchol, soit de la gp160 et de la CT.

Les compositions immunisantes sont administrées par voie nasale à des souris femelles BALB/c By J Ico. Les immunisations intra-nasales sont effectuées en 4 fois, à raison de 2x5µl dans chaque narine (i.e. 2x5 µl le matin, et 2x5µl l'après-midi).

Les quantités administrées par dose sont les suivantes : 25µg de gp160, 200µg de DCchol et 5µg de CT.

Les immunisations ont eu lieu aux jours 1,13,30 et 59.

Les prélèvements de sang ont été effectués au niveau du sinus rétro-orbitaire aux jours 42 et 75, soit respectivement après 3 et 4 immunisations.

Les sécrétions vaginales ont été prélevées après la 3^{ème} et après la 4^{ème} immunisation.

Les sécrétions rectales ont été prélevées après sacrifice des souris.

Les sécrétions nasales ont été prélevées en fin de test par un lavage naso-pharyngé.

Les dosages des IgA et IgG totales et spécifiques de la gp160 ont été effectués par ELISA dans les sérums, les sécrétions nasales, vaginales et rectales.
En résumé, des plaques NUNC Maxisorp F96 ont été sensibilisées avec des anticorps anti-IgA ou anti-IgG de souris ou avec la gp 160 MN/LAI-2 et incubées avec des dilutions sériées de sérum ou de sécrétion. Les IgA ou IgG, totales et spécifiques, ont été révélées par un conjugué peroxydase anti-IgA ou anti-IgG de souris après addition d'OPD (O-phénylènediamine-dihydrochloride).
Pour chaque sécrétion, les titres en IgA ou IgG anti-gp160 ont été divisés par les titres en IgA ou IgG totales (ratios appelés activités spécifiques normalisées, ASN) de manière à pouvoir comparer les prélèvements entre eux.

Les résultats relatifs aux anticorps sériques obtenus sont repris dans le tableau 4 ci-après où l'on voit que le DCchol est un bon adjuvant, sans toutefois atteindre l'activité de la CT, mais celle-ci a conduit à l'observation de signes de toxicité.

**Tableau 4**

| Composition vaccinale reçue | IgG anti-gp160 (µg/ml) à J41 Après la 3^{ème} immunisation | IgG anti-gp160 (µg/ml) à J79 Après la 4^{ème} immunisation |
|---|---|---|
| gp 160 (25µg) | 4,694 | 20,545 |
| gp160 (25µg) + Dcchol (200µg) | 128,698 | 172,022 |
| gp 160(25µg)+CT(5µg) | 325,091 | 430,806 |

Les résultats relatifs aux anticorps dosés dans les prélèvements naso-pharyngés sont repris dans le tableau 5 ci-après. Seules les moyennes des résultats obtenus après 4 immunisations sont indiqués.

L'analyse des activités spécifiques normalisées (ASN) montre un effet adjuvant marqué du DCchol sur les réponses IgA, malgré la grande variabilité individuelle des ratios. Par rapport à la gp160 seule, les ASN ont été multipliées d'un facteur 12 en moyenne. Ainsi, chez un certain nombre de souris, une majorité des IgA récoltées dans lavages naso-pharyngés s'est avérées spécifiques de l'antigène administré.
Au contraire, l'adjuvant selon l'art antérieur, la CT, n'a pas permis d'augmenter les réponses IgA locales, malgré son fort effet positif au niveau sérique.

**Tableau 5**

| Composition vaccinale reçue | Ratio IgA anti-gp/IgA tot (%) | Ratio IgG anti-gp/IgG (%) |
|---|---|---|
| gp160 MN/LAI-2 (25µg) | 4,76 | 0,62 |
| gp160 + DCchol (200µg) | 55,05 | 8,23 |
| gp160+ CT(5µg) | 7,28 | 7,81 |

Les résultats relatifs aux réponses mesurées dans les prélèvements vaginaux sont repris dans le tableau 6A (pour les IgG) et 6B (pour les IgA) ci-après, où ne sont reprises que les moyennes des résultats. De même que pour les prélèvements naso-pharyngés, on note ici une bonne réponse au niveau des IgG et des IgA obtenue avec les compositions selon l'invention, même si les ASN des IgA sont plus faibles que précédemment, les ASN des IgG étant elles du même ordre.

Ces résultats montrent que, grâce à une administration par voie muqueuse, on peut induire, en utilisant les compositions vaccinales selon l'invention, une réponse dans un autre compartiment muqueux très éloigné du site d'immunisation.

**Tableau 6A**

| Composition vaccinale reçue | Ratio IgG anti-gp/IgG tot (%) Après la 3^{ème} immunisation | Ratio IgG anti-gp/IgG tot (%) Après la 4^{ème} immunisation |
|---|---|---|
| gp 160 (25µg) | 0 | 0,62 |
| gp160 (25µg) + Dcchol (200µg) | 2,31 | 6,93 |
| gp 160(25µg)+CT(5µg) | 4,53 | 8,26 |

**Tableau 6B**

| Composition vaccinale reçue | Ratio IgA anti-gp/IgA tot (%) Après la 3^{ème} immunisation | Ratio IgA anti-gp/IgA tot (%) Après la 4^{ème} immunisation |
|---|---|---|
| gp 160 (25µg) | 0 | 0,95 |
| gp 160 (25µg) + Dcchol (200µg) | 7,00 | 22,32 |
| gp 160(25µg)+CT(5µg) | 1,82 | 6,75 |

Les résultats relatifs aux prélèvements rectaux sont repris dans le tableau 7 ci-après, où l'on voit que les réponses en IgA sont relativement faibles, mais où, par contre, on remarque l'efficacité des compositions vaccinales selon l'invention en ce qui concerne les IgG produites.

**Tableau 7**

| Composition vaccinale reçue | Ratio IgA anti-gp/IgA tot (%) | Ratio IgG anti-gp/IgG tot (%) |
|---|---|---|
| gp 160 (25µg) | 0 | 0,89 |
| gp160 (25µg) + Dcchol (200µg) | 0,87 | 9,02 |
| gp 160(25µg)+CT(5µg) | 0,39 | 8,91 |

### Exemple 5 : gp160 chez le macaque

On dispose, de la même façon qu'à l'exemple précédent, de gp160, de DCchol, et de toxine cholérique CT et on prépare, à partir de ces produits, des compositions immunisantes, comprenant soit de la gp160 seule, soit de la gp160 et du DCchol, soit de la gp160 et de la CT.
On veut évaluer l'efficacité chez les primates de ces compositions dans un protocole d'administration entièrement muqueux, notamment sur la réponse induite au niveau muqueux, mais également au niveau sérique.
Pour cela, on divise en 3 groupes, 12 singes Rhésus femelles, que l'on immunise simultanément par voies nasales, vaginales et rectales, à 5 reprises ( soit aux jours 1, 29, 57, 85 et 190) grâce aux compositions vaccinales suivantes :
- gp160 seule à raison de 50µg par voie et par animal,
- gp160 et DCchol à raison de 50µg de gp160 et 400µg de DCchol par voie et par animal,
- gp160 et CT à raison de 50µg de gp160 et de 50µg de CT par voie et par animal,
Les volumes administrés sont les suivants :
- pour la voie nasale : 100µl dans chaque narine,
- pour la voie vaginale : 200µl
- pour la voie rectale : 1000µl
Les prélèvements sont effectués aux dates suivantes :
- sérums : J-11/-4, 1, 29, 57, 71, 99, 184 et 203
- sécrétions vaginales : J-11, 71/78, 99/106, 184 et 203/212
- sécrétions nasales : J-11, 71, 99, 184 et 203
Les prélèvements vaginaux ont été effectués en double, à 7 ou 9 jours d'intervalle, afin d'éviter une éventuelle contamination par les menstrues.

Les dosages des IgG et IgA ont été effectués par ELISA.
De façon à s'affranchir du facteur de dilution introduit dans les sécrétions muqueuses lors des prélèvements par lavage, les titres IgA et IgG anti-gp160 ont été normalisés respectivement par les titres en IgA et IgG totales (ratios appelés activités spécifiques normalisées).

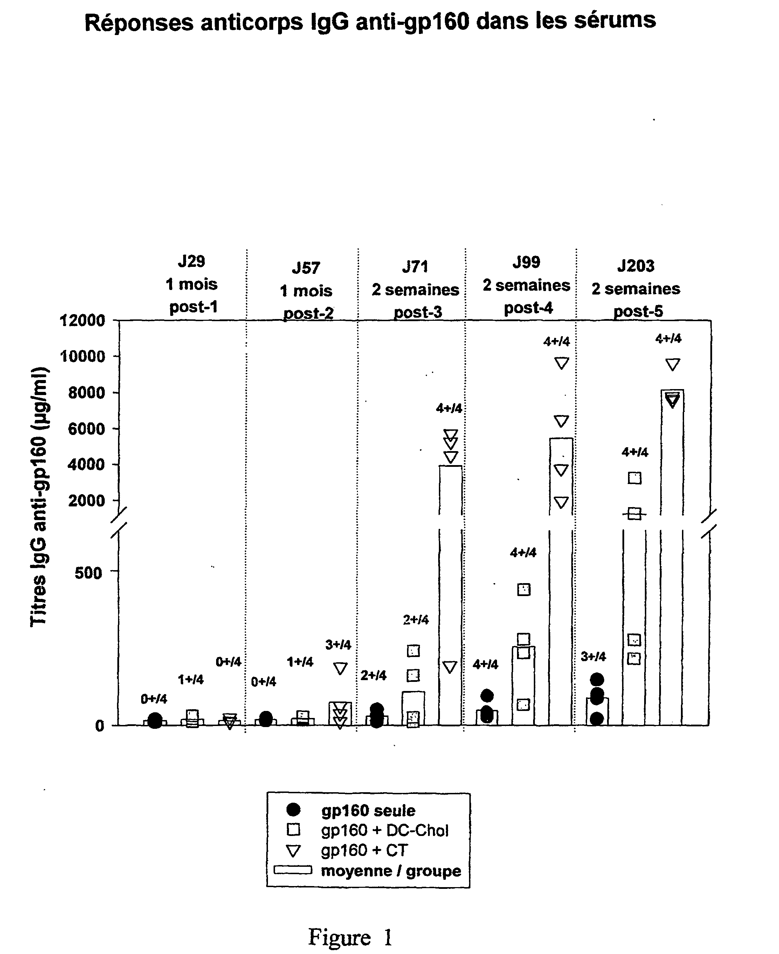

Les résultats relatifs aux réponses en IgG anti-gp160 mesurés dans les sérums sont indiqués à la Figure 1 où l'on voit nettement l'efficacité des compositions vaccinales selon l'invention, notamment après les 4^{ème} et 5^{ème} immunisations.

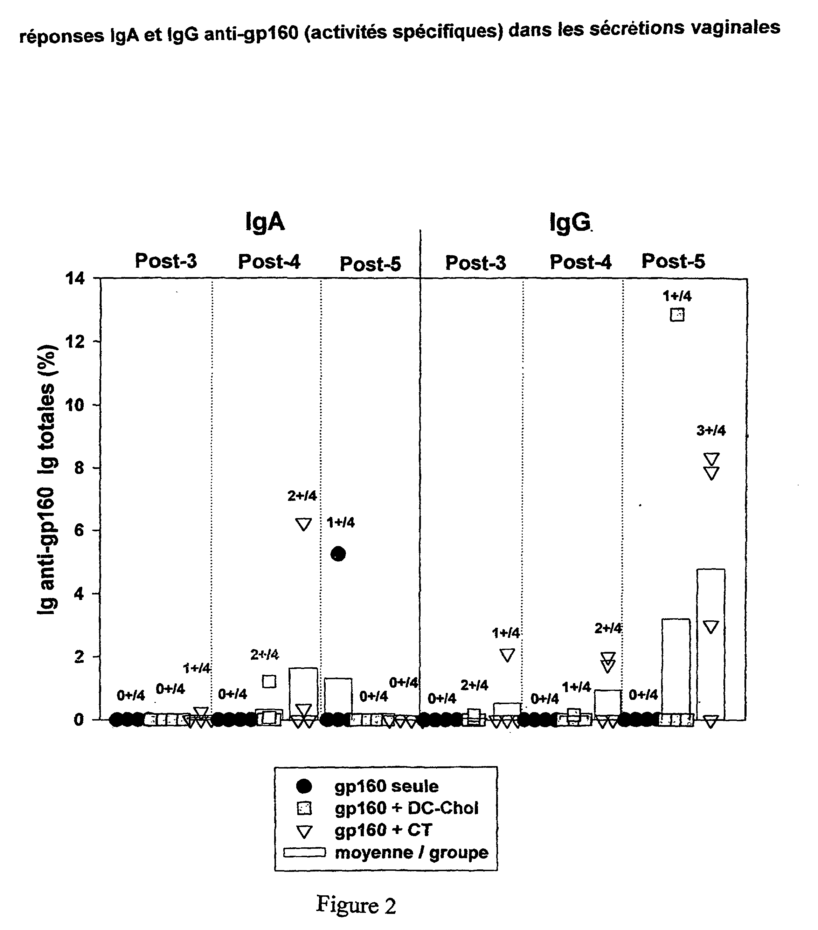

Les résultats relatifs aux réponses obtenues dans les sécrétions vaginales sont représentés sur la Figure 2 où l'on peut remarquer l'amélioration de la réponse immune induite grâce aux compositions vaccinales selon l'invention par rapport aux réponses obtenues lors de l'administration de gp160 seule.

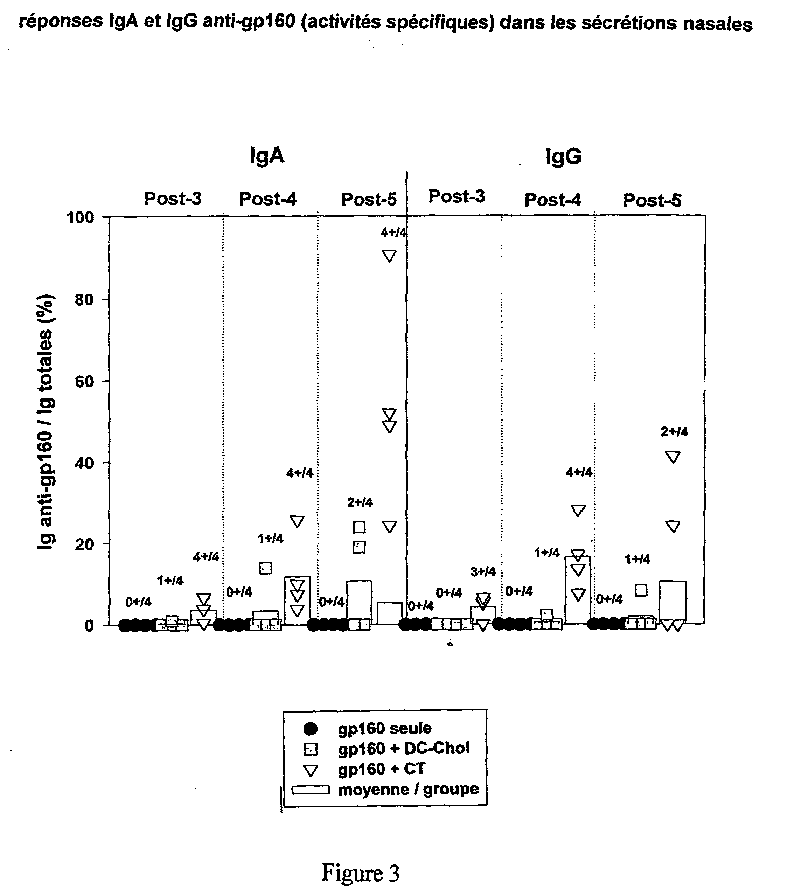

Les résultats relatifs aux réponses obtenues dans les sécrétions nasales sont représentés sur la Figure 3 où l'on note également une amélioration des réponses obtenues avec les compositions vaccinales selon l'invention par rapport aux réponses obtenues avec l'antigène seul.

### Exemple 6 : protéine Tat chez cobayes

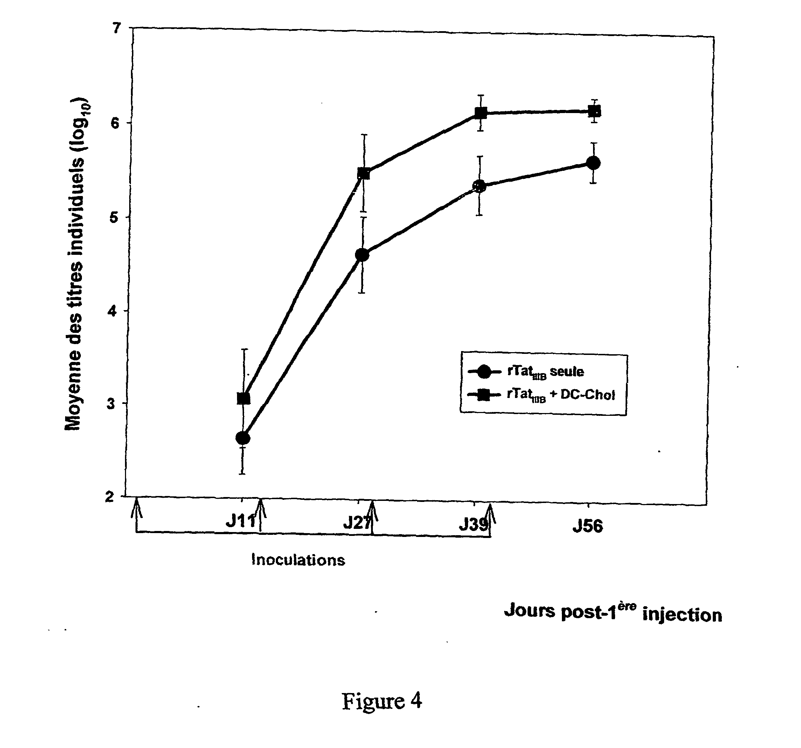

On dispose de protéine Tat carboxyméthylée, obtenue par expression chez E. coli et purification par différentes étapes de chromatographie ,puis inactivation chimique, ainsi que cela est décrit dans la demande WO99/33346 .
On prépare des compositions immunisantes comprenant soit de la protéine Tat seule en tampon PBS, soit de la protéine Tat en présence de DCchol, à raison de 500µg de DCchol par dose. Les doses vaccinantes de 1ml comprennent 50µg de protéine Tat.
On dispose ensuite de Cobayes albinos Dunkin-Hartley que l'on répartit en 2 groupes de 5 cobayes, que l'on immunise par voie intramusculaire dans la cuisse, à raison d'une injection de 0,5ml dans chaque cuisse.
Les injections sont effectuées aux jours 1, 14, 29 et 43.
Les prélèvements sériques sont effectués aux jours 11, 27, 39 et 56.
Les anticorps produits sont dosés par la méthode ELISA.
Les résultats obtenus sont présentés sur la Figure 4 où l'on voit nettement que la quantité d'anticorps anti-Tat produits est nettement plus élevée avec une composition pharmaceutique selon l'invention, qu'avec une composition ne comprenant que l'antigène seul.

### Exemple 7 :protéine Tat chez souris

On dispose de protéine Tat carboxyméthylée, obtenue par expression chez E. coli et purification par différentes étapes de chromatographie ,puis inactivation chimique, ainsi que cela est décrit dans la demande WO99/33346
On prépare des compositions immunisantes comprenant :
- de la protéine Tat à raison de 200µg/ml en tampon TRIS/NaCl
- de la protéine Tat à raison de 200µg/ml et de l'hydroxide d'aluminium en tampon TRIS/NaCl,
- de la protéine Tat à raison de 200 µg/ml et du DCchol obtenu à l'exemple 1 en tampon TRIS/NaCl,

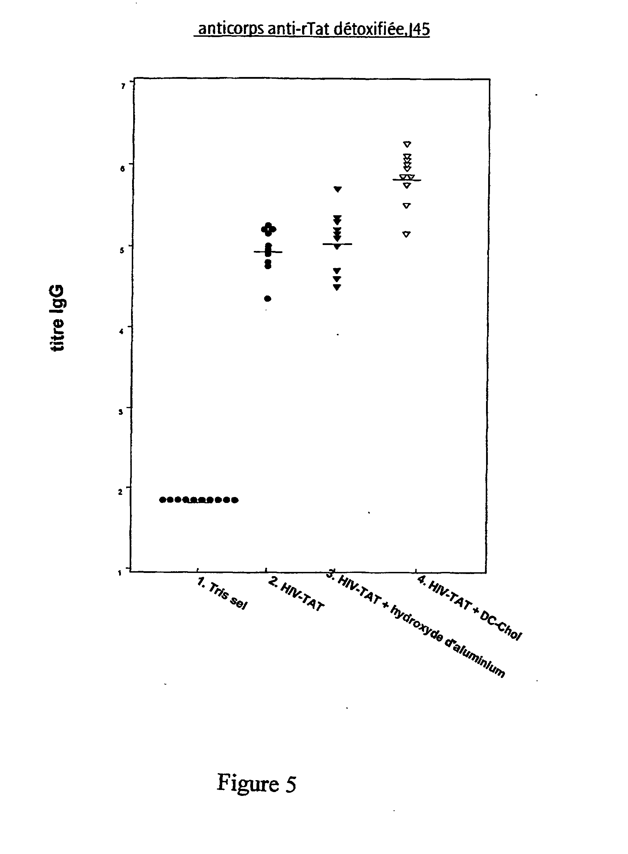

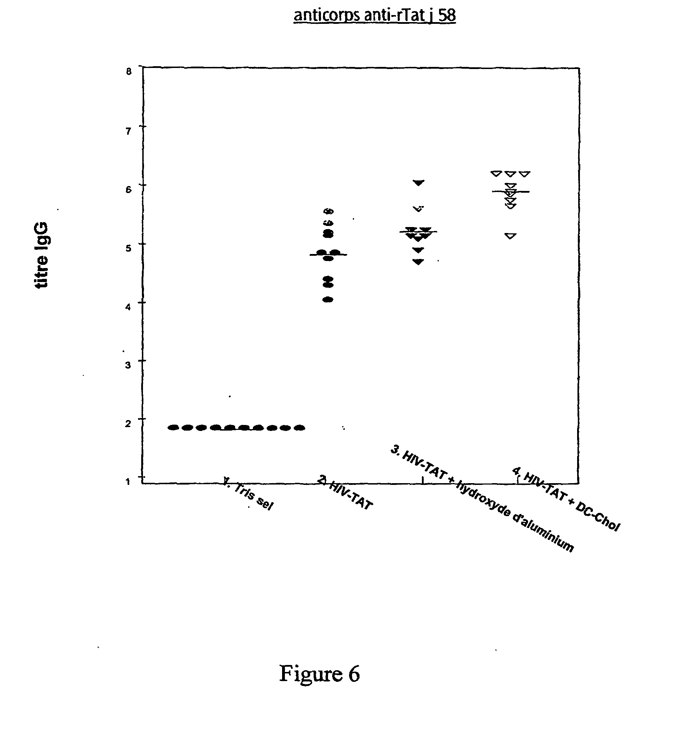

On dispose de rats Sprague Dawley, à qui on injecte les préparations immunisantes en intramusculaire, aux jours 1,8,15, 29 et 43
A chaque administration, on effectue 2 injections de 0,25ml par animal.
Des prélèvements sanguins sont effectués aux jours 45 et 58, afin de doser par ELISA les anticorps IgG anti-Tat recombinante détoxifiée.
Les résultats obtenus, exprimés en log IgG sont représentés à la Figure 5 pour les résultats à J45 et sur la Figure 6 pour les résultats à J58.
Ces résultats illustrent l'efficacité des compositions pharmaceutiques selon l'invention par rapport à des compositions vaccinales ne comprenant que l'antigène Tat.

### Exemple 8 :p24 chez la souris

On dispose de la protéine p24 qui est un antigène qui a été décrit dans la publication suivante :
*Diagnostic value of HIV-Ag testing and anti-p24 titers in HIV carriers and AIDS patients*.Roumeliotou A, Nestoridou E, Economidou I, Psarra E, Sidiri E, Choremi E, Kallinikos G, Papaevangelou G, *AIDS* 1988 Feb;2(1):64.
Cet antigène est présent à une concentration de 1mg/ml dans une solution Phosphate de Sodium 20mM, NaCl 50mM, à pH 7,5.
On dispose également d'une solution de TRIS/NaCl à 20mM de TRIS et 150mM de NaCl, à pH 8, ainsi que du DCchol obtenu selon l'exemple 1.
On prépare ainsi des compositions immunisantes ayant la composition suivantes :
- Groupe 1 : p24 (20µg/dose)
- Groupe 2 : p24 (1µg/dose)
- Groupe 3 : p24 (0,1µg/dose)
- Groupe 4 : p24 (0,01 µg/dose)
- Groupe 5 : contrôle constitué uniquement d'une solution saline
- Groupe 6 : p24 (1µg/dose) + DCchol (25µg/dose) sous forme de suspension liposomale
- Groupe 7 : p24 (1µg/dose) + DCchol (25µg/dose) en émulsion Squalène/Tween®80 dans laquelle la quantité de Squalène est de 5mg/dose et la quantité de Tween®80 est de 600µg/dose. L'émulsion est obtenue par mélange de 1g de Squalène dans 20ml de TRIS/NaCl contenant du Tween®80 et du DCchol, homogénéisé à l'ultra-turrax pendant 1min à 13500 tours/min, puis microfluidisée avant d'être filtrée. Les gouttes de l'émulsion ainsi obtenue ont une taille moyenne autour de 150nm.

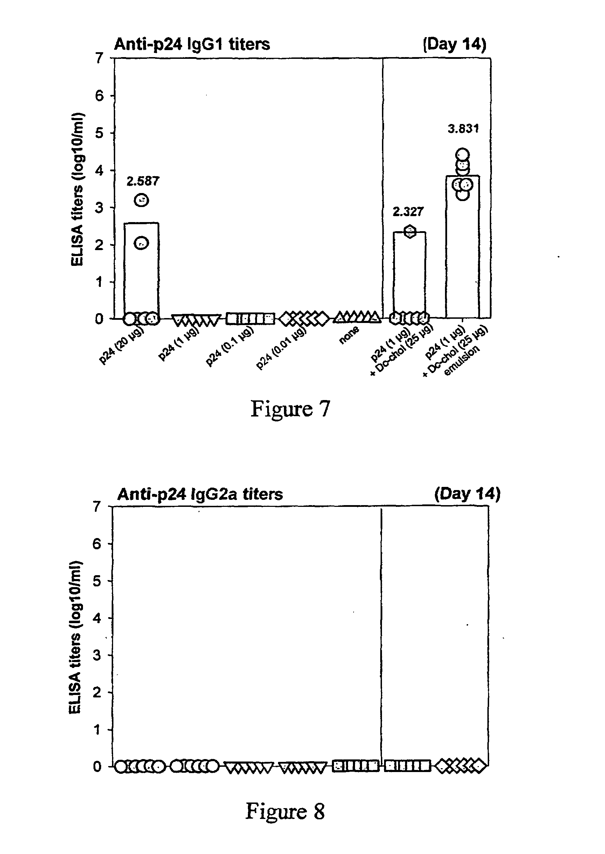

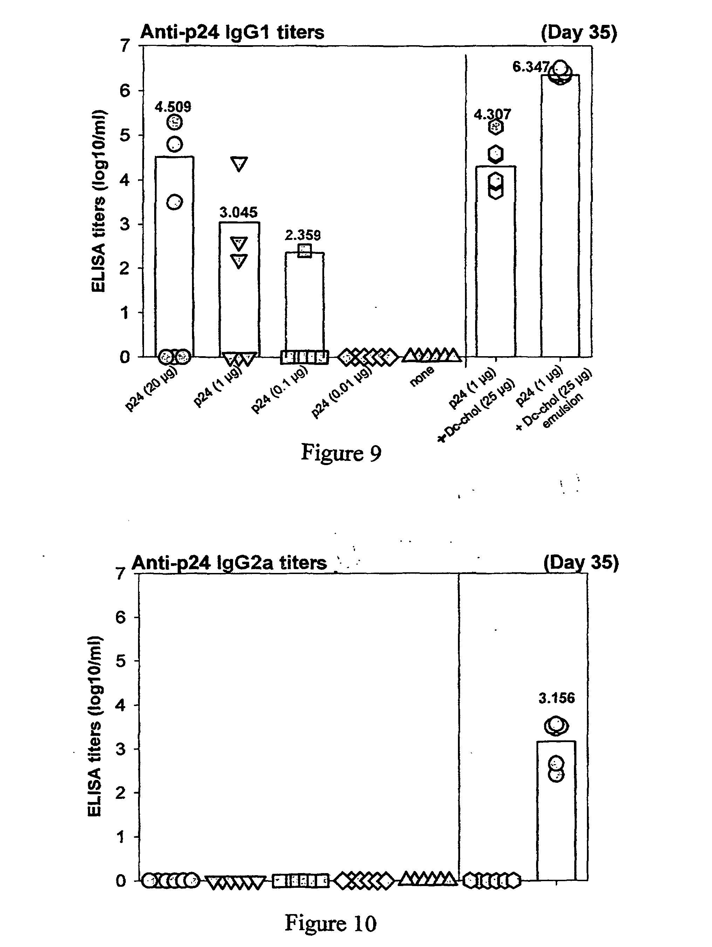

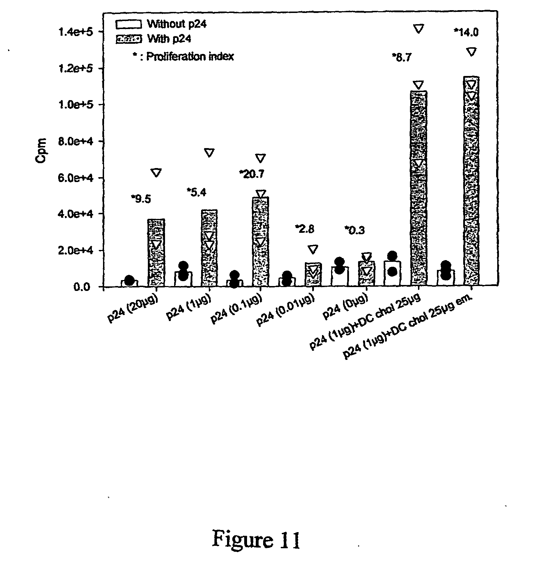

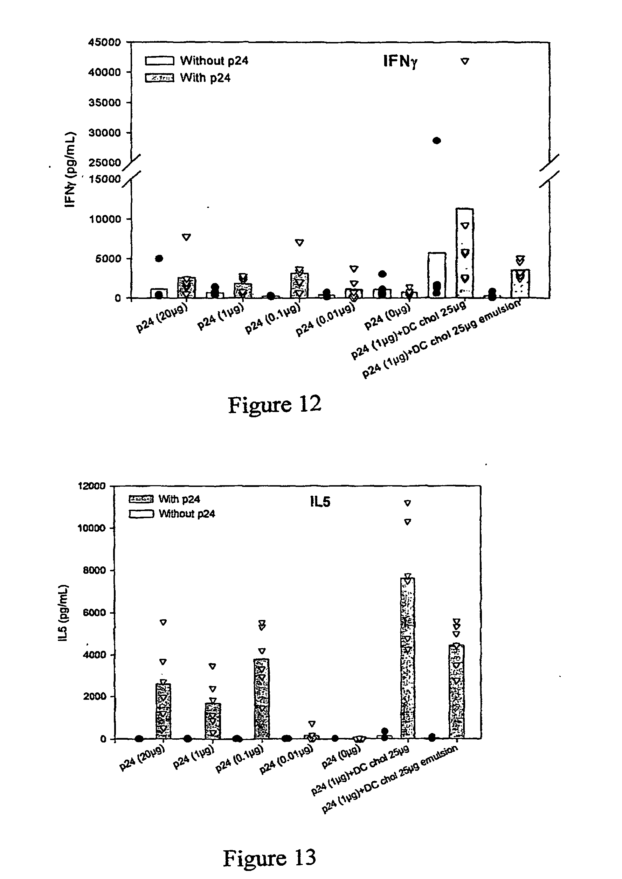

Les différentes compositions immunisantes sont administrées à des souris BALB/c réparties par groupe de 6, chaque groupe recevant une composition différente.
Chacune des souris reçoit une dose de 200µl à J1 et à J21, en sous-cutané.
Des prélèvements sont effectués à J14 et à 35 afin d'effectuer les dosages en anticorps sériques.
A J37, les souris sont sacrifiées.
Des tests de prolifération en réponse à de la protéine recombinante p24(5µg/ml) à 5 jours sont effectués.
On réalise également des dosages relatifs à la production de IL5 et d'IFNγ dans les surnageants de cellules spléniques qui sont stimulées ou non pendant 5 jours in vitro par 10mg/ml de p24 recombinante.
Les résultats obtenus sont illustrés sur les figures 7 à 13.
Ces résultats montrent que les titres en Anticorps IgG1 à J14 et à J35 (Figures 7 et 9) sont meilleurs avec les compositions pharmaceutiques selon l'invention qu'avec des compositions vaccinales comprenant uniquement l'antigène, à la même concentration d'antigène, et qu'à J35, une des compositions selon l'invention est également capable d'induire la production d'anticorps IgG2a (Figure 10), ce qui est représentatif de l'induction d'une réponse de type TH1.
En outre, les compositions vaccinales selon l'invention conduisent à un plus grand index de prolifération que les compositions comprenant uniquement l'antigène(Figure 11).
Les résultats des dosages des cytokines IFNγ et IL5 ( Figures 12 et 13) montrent que, lorsqu'on utilise une composition vaccinale selon l'invention, les quantités de cytokines produites sont également augmentées.

## Revendications

1. Utilisation d'une composition pharmaceutique comprenant au moins un antigène du VIH et du DCchol pour la préparation d'un médicament pour l'immunisation contre les infections liées au VIH, destiné à être administré par voie muqueuse.

2. Utilisation selon la revendication précédente, **caractérisée en ce que** le médicament est destiné à l'induction dans les tissus muqueux d'un mammifère, d'anticorps Ig G ou Ig A spécifiques d'un antigène du VIH.

3. Utilisation selon une des revendications précédentes, **caractérisée en ce que** l'antigène du VIH est la gp160.

4. Utilisation selon une des revendications précédentes, **caractérisée en ce que** l'antigène du VIH est la p24.

5. Utilisation selon une des revendications précédentes, **caractérisée en ce que** l'antigène du VIH est la protéine Tat.

6. Utilisation selon une des revendications précédentes, **caractérisée en ce que** la composition pharmaceutique comprend en outre un oligonucléotide immunostimulant.

7. Utilisation selon une des revendications précédentes, **caractérisée en ce que** la composition pharmaceutique se présente sous forme d'émulsion.

## Patentansprüche

1. Verwendung einer pharmazeutischen Zusammensetzung, die wenigstens ein HIV-Antigen und DCchol umfasst, für die Herstellung eines Medikaments für die Immunisierung gegen die mit HIV verbundenen Infektionen, das über die Schleimhäute verabreicht werden soll.

2. Verwendung gemäß dem vorhergehenden Anspruch, **dadurch gekennzeichnet, dass** das Medikament zur Induktion von Ig G- oder Ig A-Antikörpern, die für ein HIV-Antigen spezifisch sind, in den Schleimhautgeweben eines Säugers bestimmt ist.

3. Verwendung gemäß einem der vorhergehenden Ansprüche, **dadurch gekennzeichnet, dass** das HIV-Antigen gp160 ist.

4. Verwendung gemäß einem der vorhergehenden Ansprüche, **dadurch gekennzeichnet, dass** das HIV-Antigen p24 ist.

5. Verwendung gemäß einem der vorhergehenden Ansprüche, **dadurch gekennzeichnet, dass** das HIV-Antigen das Protein Tat ist.

6. Verwendung gemäß einem der vorhergehenden Ansprüche, **dadurch gekennzeichnet, dass** die pharmazeutische Zusammensetzung außerdem ein immunstimulierendes Oligonukleotid umfasst.

7. Verwendung gemäß einem der vorhergehenden Ansprüche, **dadurch gekennzeichnet, dass** die pharmazeutische Zusammensetzung in Form einer Emulsion vorliegt.

## Claims

1. Use of a pharmaceutical composition comprising at least one HIV antigen and DCchol, for preparing a medicinal product for immunization against HIV-related infections, intended to be administered mucosally.

2. Use according to the preceding claim, **characterized in that** the medicinal product is for inducing, in the mucosal tissues of a mammal, IgG or IgA antibodies specific for an HIV antigen.

3. Use according to either of the preceding claims, **characterized in that** the HIV antigen is gp160.

4. Use according to one of the preceding claims, **characterized in that** the HIV antigen is p24.

5. Use according to one of the preceding claims, **characterized in that** the HIV antigen is the Tat protein.

6. Use according to one of the preceding claims, **characterized in that** the pharmaceutical composition additionally comprises an immunostimulant oligonucleotide.

7. Use according to one of the preceding claims, **characterized in that** the pharmaceutical composition is present in the form of an emulsion.
